**Europäisches Patentamt**

**European Patent Office**

**Office européen des brevets**

(19)

(11) Veröffentlichungsnummer: **0 075 753
B1**

(12) # EUROPÄISCHE PATENTSCHRIFT

(45) Veröffentlichungstag der Patentschrift:
**02.01.85**

(51) Int. Cl.³: **A 41 D 13/12,** A 61 M 25/02,
A 61 B 19/00, A 61 M 5/00

(21) Anmeldenummer: **82108228.6**

(22) Anmeldetag: **07.09.82**

(54) Weste für kontinuierliche Sondenernährung.

(30) Priorität: **28.09.81 DE 3138568**

(43) Veröffentlichungstag der Anmeldung:
**06.04.83 Patentblatt 83/14**

(45) Bekanntmachung des Hinweises auf die Patenterteilung:
**02.01.85 Patentblatt 85/1**

(84) Benannte Vertragsstaaten:
**AT BE CH DE FR GB IT LI LU NL SE**

(56) Entgegenhaltungen:
**DE - A - 2 832 800
US - A - 3 726 280
US - A - 3 895 629
US - A - 4 150 672
US - E - 27 348**

(73) Patentinhaber: **Fresenius AG, Gluckensteinweg 5,
D-6380 Bad Homburg (DE)**

(72) Erfinder: **Selle, Dorothea, Stettiner Strasse 23,
D-6370 Oberursel 6 (DE)**

(74) Vertreter: **KUHNEN & WACKER Patentanwaltsbüro,
Schneggstrasse 3-5 Postfach 1729, D-8050 Freising (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Weste für die kontinuierliche Sondenernährung mit einem Vorder- und Rückenteil, die über zwei Schulterteile sowie an ihren Rändern zusammengehalten werden und bei denen wenigstens ein Teil zum An- und Ausziehen offenbar ist, wobei auf dem Vorderteil Taschen zur Aufnahme einer Pumpe und wenigstens eines Behälters für die Nährlösung vorgesehen sind.

Die kontinuierliche Sondenernährung stellt eine Alternative zur Infusionstherapie dar, die üblicherweise im Krankenhaus durchgeführt wird, so daß der Patient, obwohl er häufig mobil ist, ans Bett gefesselt ist. Dies hatte zur Folge, daß erhebliche Kosten für die Behandlung des Patienten und die Belegung des Krankenhauses anfielen, obwohl der Patient auch ambulant hätte behandelt werden können. Die Ernährung durch Infusion ist insbesondere bei Problempatienten und bei Kindern problematisch, die häufig nicht ans Bett gefesselt werden können und daher diese Ernährungsart als lästig empfinden.

Diese Problematik kann durch eine kontinuierliche enterale Sondenernährung gelöst werden, bei der dem Patienten Flüssignahrung über eine schlauchartige Ernährungssonde zugeführt wird. Dabei wird die Nährlösung in einem Beutel oder Behälter vorgelegt, dessen Ausgang mit der Ernährungssonde über eine Leitung in Verbindung steht. Die Leitung wird durch eine üblicherweise als Rollenpumpe ausgeführte Pumpe durchgeführt, die die Nährlösung mit einer bestimmten kontinuierlichen Pumpleistung durch die Ernährungssonde fördert. Von der Pumpe erstreckt sich die Ernährungssonde durch eine Körperöffnung des Patienten, beispielsweise durch die Nase, in den Körper des Patienten, vorteilhafterweise in den Magen-Darmtrakt.

Die kontinuierliche enterale Ernährung ist insbesondere dann angezeigt, wenn folgende Krankheitszustände auftreten:

schwere Maldigestions- und Malabsorptionssyndrome,
postoperativ nach Magen- und Darmresektionen;
hyperkatabole Zustände, beispielsweise Carcinom.

Bei solchen Zuständen kann die frühzeitige enterale Ernährung nicht nur stationär, sondern auch ambulant durchgeführt werden, da eine exakte Dosierung möglich ist, der Ernährungsaufbau bis zur Adaption des Darmes an die erforderliche Nährstoffzufuhr kontrolliert werden kann, die Nährstoffe gegenüber der diskontinuierlichen Sondenernährung günstiger ausgenutzt werden, durch die stetige Ernährung eine höhere Energiezufuhr möglich ist, nur eine geringe Komplikationsrate auftritt und insbesondere bei Diabetikern eine ausgeglichene Stoffwechselführung erreicht werden kann.

Da die Patienten das enterale Ernährungssystem körperseitig tragen, um mobil zu sein, mußten entsprechende Vorkehrungen getroffen werden, damit diese Voraussetzungen erfüllt werden können.

Eine bekannte Weste für die parenterale Ernährung lehnt sich an eine typische Anzugsweste an, deren Vorderteil kontinuierlich in die beiden Schulterteile übergeht und deren Rückenteil in konventioneller Ausführung den gesamten Rücken abdeckt. Die Weste selbst ist aus einem netzartigen Kunststoffmaterial gefertigt, das zwar die Hautatmung ermöglicht, nicht jedoch besonders schwitzfreundlich ist, und kann in der Mitte des Vorderbereichs durch einen Reißverschluß geöffnet werden. Da Seiten- und Rückenteil an ihren im Unterarmbereich liegenden Rändern fest miteinander verbunden sind, muß die Weste an unterschiedliche Körpergrößen entsprechend der Konfektionsgröße angepaßt werden, so daß hierdurch eine umfangreiche Westenproduktion in Abhängigkeit von der Statur des Patienten nötig ist.

Weiterhin sind bei der bekannten Weste, die in ihrer gesamten Breite über den Brustbereich des Patienten geführt ist, Beuteltaschen direkt im Brustbereich angeordnet. Eine derartige Anordnung trägt zum einen auf und ist daher nicht sonderlich bequem und behindert zum anderen die Patienten, insbesondere weibliche Patienten, in ihrer Bewegungsfreiheit. Überdies herrscht in dieser bekannten Weste eine ungünstige Gewichtsverteilung, die sowohl auf die unterschiedliche Höhe der anzubringenden Teile (Pumpe und Behälter) als auch an deren Anordnung zur Körperlängsachse zurückzuführen ist.

Der Erfindung liegt daher die Aufgabe zugrunde, eine Weste der eingangs erwähnten Art zu schaffen, die einen weiten Konfektionsbereich überspannt und dennoch dem Patienten eine größtmögliche Bewegungsfreiheit gestattet und andrerseits eine ausgewogene Gewichtsverteilung besitzt.

Erfindungsgemäß wird die Aufgabe dadurch gelöst, daß das Vorderteil als Bauchgurt ausgebildet ist, der die Taschen zur Aufnahme der Pumpe und des Behälters nebeneinander angeordnet aufweist und in seiner Weite verstellbar ist.

Gegenüber der bekannten Weste weist die erfindungsgemäße Weste zunächst den Vorteil auf, daß das Vorderteil in Form eines Gurtes ausgeführt ist, der den Bauch des Patienten übergreift. Dieser Gurt setzt unterhalb der Brust des Patienten an und wird jeweils um den Bauch des Patienten zum Rückenteil geführt. Dieses Rückenteil kann als übliches Rückenteil ausgebildet sein oder aber es kann vorteilhafterweise ebenfalls als Gurt mit den gleichen Größenverhältnissen wie das Vorderteil ausgebildet sein.

Um seinen sicheren Sitz der Weste zu gewährleisten, ist der Bauchgurt mit zwei in ihrer Länge verstellbaren Schultergurten versehen, die über die Schulter des Patienten reichen und mit dem Rückenteil verbunden sind. Durch die Veränderbarkeit der Gurtlänge wird gewährleistet, daß die Weste paßgenau an unterschiedliche Oberkörperlängen angepaßt werden kann.

Als besonders vorteilhaft hat sich erwiesen, daß der Bauchgurt an den Seitenrändern, d. h. am seitlichen Übergang zwischen Vorder- und Rückenteil, in seiner Weite verändert werden kann, so daß eine einzige Weste der Neuerung die Damengrößen von 36—42 und die Herrengrößen bis zur Größe 52 abdeckt. Diese Erweiterungsfähigkeit wird in einer bevorzugten Ausführungsform dadurch erreicht, daß wenigstens ein, vorzugsweise auf jeder Seite ein Klettverschluß angebracht ist, der kontinuierlich an unterschiedliche Bundweiten angepaßt werden kann.

Weiterhin sind die Taschen zur Aufnahme der Pumpe und der Beutel oder Behälter für die Nährlösung nebeneinander auf dem Gurt so angeordnet, daß eine im wesentlichen gleichmäßige Gewichtsverteilung bezogen auf die Zentralkörperachse des Patienten erhalten wird. Überdies liegt das Gewicht der Pumpe und des Beutels in Bauchhöhe vor, was subjektiv wesentlich angenehmer erscheint, als auf der Brust zu tragende Gewichte.

Weitere Vorteile, Ausführungsformen und Einzelheiten ergeben sich aus den Unteransprüchen sowie aus der folgenden Beschreibung unter Bezugnahme auf die Zeichnung. Es zeigt

Fig. 1 die erfindungsgemäße Weste perspektivisch,

Fig. 2 die Weste im aufgeklappten Zustand in der Draufsicht, wobei die dem Körper zugewandten Flächen ersichtlich sind, und

Fig. 3 die Weste im aufgeklappten Zustand in der Draufsicht, wobei die im getragenen Zustand sichtbaren Flächen ersichtlich sind.

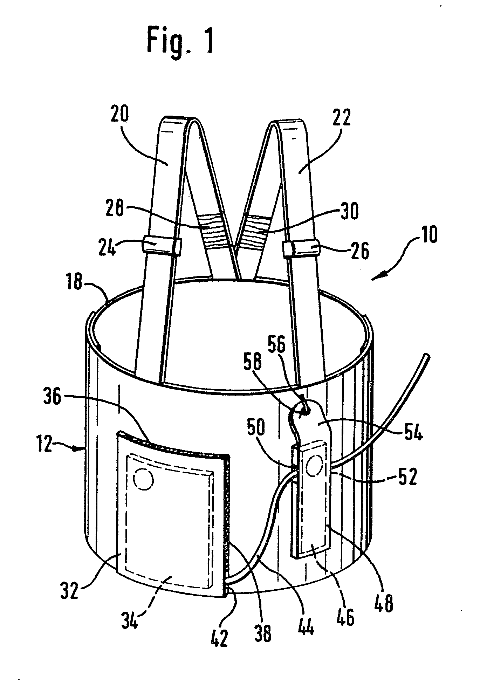

Aus Fig. 1 ist die erfindungsgemäße Weste 10 in perspektivischer Darstellung ersichtlich. Diese Weste 10 besteht im wesentlichen aus einem Gurtteil 12, das sich in ein Vorderteil 14 und in ein Rückenteil 16 unterteilt. Am oberen Rand 18 des Gurtteils sind die Schultergurte 20 und 22 befestigt, die auf dem Rückenteil 16 V-förmig zusammenlaufen und auf dem Vorderteil 14 in einem bestimmten Abstand voneinander befestigt sind. Beide Schultergurte 20 und 22 sind in Höhe des Gurtspanners 24 und 26 vorzugsweise unterteilt und können in ihrer Länge mit Hilfe dieses Gurtspanners 23 und 26 verändert werden, so daß eine hohe Paßgenauigkeit auch bei unterschiedlichen Oberkörperlängen erzielt werden kann. Diese Genauigkeit kann andererseits auch mit nicht unterteilten Gurten erreicht werden. Zur Anpassung der Schultergurte 20 und 22 an Körperbewegungen ist im Gurt jeweils ein Gummizweig 28 und 30 vorgesehen, der ein elastisches Anliegen des Schultergurts am Oberkörper des Patienten gewährleistet.

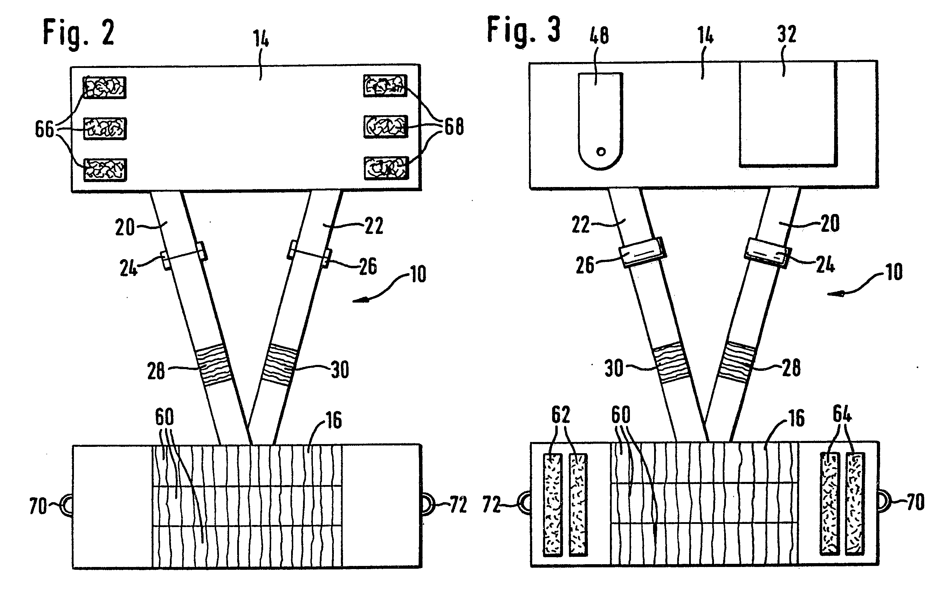

Die vordere Außenoberfläche des Vorderteils 14, die aus Fig. 1 und 3 ersichtlich ist, weist eine Tasche 32 auf, in die ein Beutel 34 einlegbar ist, der die gewünschte Nährlösung enthält. Die Tasche ist entlang ihrer Seitenränder durch die Reißverschlüsse 36 und 38 zu öffnen, so daß der Beutel 34 leicht handhabbar ist und ohne weiteres entnommen werden kann. Dieser Beutel 34 besitzt üblicherweise ein Fassungsvolumen von etwa 1 Ltr., kann jedoch aber auch durch eine entsprechende Faltung weniger Nährflüssigkeit aufnehmen, was für kleinere Taschen beispielsweise bei Kinderwesten von Bedeutung ist.

Die Tasche 32 weist unterhalb des Reißverschlusses 38 eine Öffnung 42 auf, durch die die Sondenleitung 44 mit dem Beutel 34 verbunden werden kann. Die Sondenleitung 44 wird in eine Pumpe 46 eingelegt, die üblicherweise als Rollenpumpe ausgebildet ist. Diese Pumpe 46 befindet sich in einer Tasche 48, die vorzugsweise zur Schalldämmung und Stoßsicherung auf ihrer Außenseite mit einer entsprechenden Schicht gedämmt ist. Sie weist weiterhin einen seitlichen Schlitz 50 und 52 auf, durch den die Sondenleitung in die Pumpe eingeführt werden kann.

Zur besseren Halterung der Pumpe 46 ist die Tasche 48 über eine Lasche 54 und einen Klappverschluß 56 verschließbar, der durch eine entsprechende Öffnung 58 in der Lasche 54 durchgeschoben und umgeklappt werden kann.

Das Rückenteil 16 weist zunächst horizontal verlaufende Gummizüge auf, die sich an eine Bewegung des Patienten elastisch anpassen. Weiterhin befinden sich auf der im geöffneten Zustand sichtbaren Außenseite des Rückenteils 16 streifenförmig verlaufende Klettverschlußteile 62 und 64, wobei die Streifen in Körperlängsrichtung verlaufen.

Die komplementären Klettverschlußteile 66 und 68 befinden sich auf der Innenseite des Vorderteils 14 und verlaufen im wesentlichen horizontal. Sie sind ebenfalls streifenförmig ausgebildet und können infolgedessen durch Zusammenwirken mit den jeweiligen Klettverschlußteilen 62 und 64 fest miteinander verbunden werden. Im Verbindungszustand sind diese Streifen im wesentlichen rechtwinklig zueinander angeordnet und geben somit einen optimalen Sitz. Natürlich läßt sich die Bundweite sowie die Anordnung von Vorder- und Rückenteil zueinander beliebig innerhalb der vorgegebenen Maße anordnen, so daß weitgehend eine Unabhängigkeit von Konfektionsgrößen gegeben ist.

Anstelle der Klettverschlüsse kann natürlich auch jedes andere übliche Befestigungsmittel, wie Reißverschlüsse, Haken u. dgl. eingesetzt werden.

In einer speziellen Ausführungsform sind weiterhin entlang der Seitenränder des Rückenteils 16 Ringe 70 und 72 vorgesehen, durch die ein Befestigungsband durchgezogen werden kann, sofern eine weitere zusätzliche Befestigung gewünscht ist. Dieses Band kann dabei auf den Rückenteil befestigt sein.

Eine weitere Ausführungsform sieht vor, daß das Rückenteil 16 bis an die Schultern hochgezogen ist. In dieser Ausführung enden natürlich die Schultergurte 20 und 22 in Schulterhöhe und erstrecken sich nicht mehr über die Schulter auf den Rücken.

Hinzuzufügen ist, daß die Klettverschlüsse 62—68 in vorteilhafterweise deswegen streifenförmig unterteilt sind, weil hierdurch die Formsteife dieses Verschlusses aufgehoben wird und dadurch eine bequemere Handhabbarkeit der Weste 10 erreicht wird.

Die Weste selbst ist aus einem luftdurchlässigen und kochfesten Material, vorzugsweise aus Baumwolle gefertigt, die infolge dieser Eigenschaften eine angenehme Trageweise garantiert. Üblicherweise weist das Gurtteil 12 für Erwachsene eine Breite von ca. 15—30, vorzugsweise etwa 20—23 cm auf, so daß es bequem zwischen dem unteren Rippenbogen und der Hüfte getragen werden kann. Durch diese Anordnung wird der natürliche Bewegungsablauf nicht beeinträchtigt. Weiterhin ist die gesamte Armpartie freigehalten, so daß eine uneingeschränkte Bewegungsmöglichkeit der Arme gegeben ist. Durch die verschiedenen Gummizüge wird eine individuelle Anpassung der Weste ermöglicht.

Infolgedessen können sitzende wie stehende Tätigkeiten ausgeführt werden. Außerdem fällt die Weste durch ihre unauffällige, unter der Oberbekleidung versteckte Trageweise nicht auf, was die Rehabilitation der Patienten psychisch günstig beeinflußt.

**Patentansprüche**

1. Weste für die kontinuierliche Sondenernährung mit einem Vorder- und Rückenteil, die über zwei Schulterteile sowie an ihren Rändern zusammengehalten werden, bei denen wenigstens ein Teil zum An- und Ausziehen offenbar ist, wobei auf dem Vorderteil Taschen zur Aufnahme einer Pumpe und wenigstens eines Behälters für die Nährlösung vorgesehen sind, dadurch gekennzeichnet, daß das Vorderteil (14) als Bauchgurt ausgebildet ist, der die Taschen (32, 48) zur Aufnahme der Pumpe (46) und des Behälters (34) nebeneinander angeordnet aufweist und in seiner Weite verstellbar ist.

2. Weste nach Anspruch 1, dadurch gekennzeichnet, daß das Vorderteil (14) und das Rückenteil (16) durch wenigstens einen Klettverschluß verbindbar sind.

3. Weste nach Anspruch 1 oder 2, dadurch gekennzeichnet, daß der Klettverschluß in Streifen unterteilt ist und daß Verschlußteile (62, 64), die sich auf der Außenseite des Rückenteils befinden im wesentlichen rechtwinklig zu Verschlußteilen (66, 68) die sich auf der Innenseite des Vorderteils befinden, angeordnet sind.

4. Weste nach einem der Ansprüche 1—3, dadurch gekennzeichnet, daß das Rückenteil (16) wenigstens einen Gummizug (60) aufweist, der horizontal elastisch ist.

5. Weste nach einem der Ansprüche 1—4, dadurch gekennzeichnet, daß die Schultergurte (20, 22) V-förmig an dem Rückenteil (16) befestigt sind und elastisch über Gummizüge (28, 30) geführt sind.

6. Weste nach Anspruch 5, dadurch gekennzeichnet, daß die Schultergurte (20, 22) über Gurtspanner (24, 26) in ihrer Länge veränderbar sind.

7. Weste nach einem der Ansprüche 1—6, dadurch gekennzeichnet, daß die Tasche (32) an ihren Seitenrändern mit Reißverschlüssen (36, 38) öffenbar ist.

8. Weste nach einem der Ansprüche 1—7, dadurch gekennzeichnet, daß die Tasche (32) eine Öffnung (42) für die Sonde (44) aufweist.

9. Weste nach einem der Ansprüche 1—8, dadurch gekennzeichnet, daß die Pumpe (46) in der Tasche (48) mittels einer Lasche (54) und eines Klappverschlusses (56) fest angeordnet ist.

10. Weste nach einem der Ansprüche 1—9, dadurch gekennzeichnet, daß die Tasche (48) seitliche Schlitze (50, 52) zur Einführung der Sonde (44) in die Pumpe (46) aufweist.

11. Weste nach einem der Ansprüche 1—10, dadurch gekennzeichnet, daß das Vorderteil (14) und das Rückenteil (16) im wesentlichen die gleiche Form aufweisen und an ihren Seitenrändern durch zwei Klettverschlüsse miteinander verbindbar sind.

12. Weste nach einem der Ansprüche 1—11, dadurch gekennzeichnet, daß die Weste aus einem luftdurchlässigen und kochfesten Material, vorzugsweise Baumwolle besteht.

**Claims**

1. Vest for continuous tubing feeding having a ventral part and a dorsal part, which are held together by two shoulder parts and at their edges, in which at least one part can be opened for putting on and taking off, there being provided on the ventral part pockets to receive a pump and at least one container for the nutrient solution, characterised in that the ventral part (14) is designed as an abdominal belt which has the pockets (32, 48) to receive the pump (46) and the container (34) arranged next to each other and the width of which can be adjusted.

2. Vest according to Claim 1, characterised in that the ventral part (14) and the dorsal part (16) can be joined together by at least one touch-and-close fastener.

3. Vest according to Claim 1 or 2, characterised in that the touch-and-close fastener is divided into strips and in that the fastener parts (62, 64) which are located on the outside of the dorsal part, are arranged essentially at right angles to the fastener parts (66, 68) which are located on the inside of the ventral part.

4. Vest according to one of Claims 1—3, characterised in that the dorsal part (16) has at least one piece of elastic webbing (60) which is horizontally elastic.

5. Vest according to one of Claims 1—4, characterised in that the shoulder straps (20, 22) are attached in the shape of a V to the dorsal part (16) and are elasticated by pieces of elastic webbing (28, 30).

6. Vest according to Claim 5, characterised in that the lengths of the shoulder straps (20, 22) can be changed by belt tensioners (24, 26).

7. Vest according to one of Claims 1—6, characterised in that the edges of the pocket (32) can be opened by zip fasteners (36, 38).

8. Vest according to one of Claims 1—7, characterised in that the pocket (32) has an opening (42) for the tube (44).

9. Vest according to one of Claims 1—8, characterised in that the pump (46) in fixed in the pocket (48) by means of a flap (54) and a flap fastener (56).

10. Vest according to one of Claims 1—9, characterised in that the pocket (48) has slits (50, 52) in the side for introduction of the tube (44) into the pump (46).

11. Vest according to one of Claims 1—10, characterised in that the ventral part (14) and the dorsal part (16) have essentially the same shape and the lateral edges of which can be joined together by means of two touch-and-close fasteners.

12. Vest according to one of Claims 1—11, characterised in that the vest is composed of a material which is permeable to air and resistant to boiling, preferably cotton.


**Revendications**

1. Gilet pour l'alimentation continue par sonde comportant une partie avant et une partie arrière tenues ensemble par deux parties d'épaules ainsi qu'en leurs bords, dont a moins une partie peut être ouverte pour enfiler et enlever le gilet, le devant portant des poches pour recevoir une pompe et au moins un récipient pour la solution nutritive, caractérisé par le fait que le devant (14) est formé en tant que ceinture ventrale sur laquelle les poches (32, 48) pour recevoir la pompe (46) et le récipient (34) sont juxtaposés et est réglable en largeur.

2. Gilet selon la revendication 1 caractérisé par le fait que le devant (14) et le dos (16) peuvent être raccordés par au moins une fermeture velcro.

3. Gilet selon l'une des revendications 1 ou 2 caractérisé par le fait que la fermeture velcro est subdivisée en bandes et que les parties de fermeture (62, 64) se trouvant sur le côté extérieur du dos sont disposées substantiellement à angle droit par rapport aux pièces de fermetures (66, 68) qui se trouvent sur la face interne du devant.

4. Gilet selon l'une des revendications 1 à 3 caractérisé par le fait que le dos (16) comporte au moins un élastique en coulisse (60) qui est élastique en horizontale.

5. Gilet selon l'une des revendications 1 à 4 caractérisé par le fait que les bretelles (20, 22) sont fixées en V au dos (16) et sont adaptables par des élastiques en coulisse (28, 30).

6. Gilet selon la revendication 5 caractérisé par le fait que les bretelles (20, 22) peuvent être variées en longueur par l'intermédiaire de tendeurs (24, 26).

7. Gilet selon l'une des revendications 1 à 6 caractérisé par le fait que la poche (32) peut être ouverte par des fermetures éclair (36, 38) le long de ses coutures latérales.

8. Gilet selon l'une des revendications 1 à 7 caractérisé par le fait que la poche (32) a une ouverture (42) pour la sonde (44).

9. Gilet selon l'une des revendications 1 à 8 caractérisé par le fait que la pompe (46) est fermement logée dans la poche (48) au moyen d'une bride (54) et d'une fermeture rabattable (56).

10. Gilet selon l'une des revendications 1 à 9 caractérisé par le fait que la poche (48) a des fentes latérales (50, 52) pour l'introduction de la sonde (44) dans la pompe (46).

11. Gilet selon l'une des revendications 1 à 10 caractérisé par le fait que le devant (14) et le dos (16) ont substantiellement la même forme et sont raccordables en leurs bords latéraux par deux fermetures velcro.

12. Gilet selon l'une des revendications 1 à 11 caractérisé par le fait que le gilet est en un matériel poreux résistant à la cuisson, de préférence en coton.

# Fig. 1

Fig. 2

Fig. 3

0 075 753